(19) Europäisches Patentamt
European Patent Office
Office européen des brevets

(11) Veröffentlichungsnummer: **0 044 989**
**B1**

(12) # EUROPÄISCHE PATENTSCHRIFT

(45) Veröffentlichungstag der Patentschrift: **25.05.83**

(51) Int. Cl.³: **C 07 D 243/38**, C 07 D 401/06, A 61 K 31/55

(21) Anmeldenummer: **81105421.2**

(22) Anmeldetag: **11.07.81**

(54) **Neue, in 5-Stellung substituierte 5,10-Dihydro-11H-dibenzo (b,e)(1,4)diazepin-11-one, Verfahren zu ihrer Herstellung und diese Verbindungen enthaltende Arzneimittel.**

(30) Priorität: **24.07.80 DE 3028001**

(43) Veröffentlichungstag der Anmeldung: **03.02.82 Patentblatt 82/5**

(45) Bekanntmachung des Hinweises auf die Patenterteilung: **25.05.83 Patentblatt 83/21**

(84) Benannte Vertragsstaaten: **AT BE CH DE FR IT LI LU NL SE**

(56) Entgegenhaltungen:
**DE-A-1 931 487**
**DE-A-2 022 790**
**DE-C-1 795 176**

(73) Patentinhaber: **Dr. Karl Thomae GmbH, Postfach 720, D-7950 Biberach (Riss) (DE)**

(72) Erfinder: **Schmidt, Günther, Dr. Dipl.-Chem, Johann-Sebastian-Bach-Strasse 27, D-7950 Biberach 1 (DE)**
Erfinder: **Bergamaschi, Mario, Prof. Dr., Via Missori, 14, I-20052 Monza (IT)**

**EP 0 044 989 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue, in 5-Stellung substituierte 5,10-Dihydro-11H-dibenzo[b,e] [1,4]diazepin-11-one, Verfahren zu ihrer Herstellung und diese Verbindungen enthaltende Arzneimittel

Die Erfindung betrifft neue, in 5-Stellung substituierte 5,10-Dihydro-11H-dibenzo[b,e] [1,4]diazepin-11-one der allgemeinen Formel I,

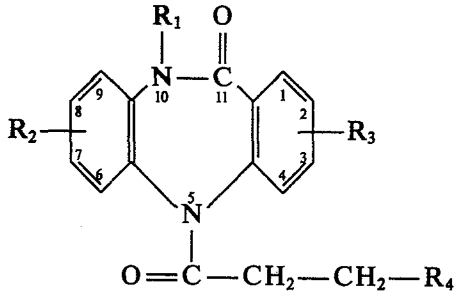

(I)

sowie deren physiologisch verträgliche Salze mit anorganischen und organischen Säuren, Verfahren zu ihrer Herstellung und diese Verbindungen enthaltende Arzneimittel.

In der obigen allgemeinen Formel I bedeuten

$R_1$ ein Wasserstoffatom, die Methyl- oder Ethylgruppe,
$R_2$ ein Wasserstoff- oder Chloratom,
$R_3$ ein Wasserstoff- oder Chloratom und
$R_4$ die Pyrrolidino-, Piperidino-, 2-Methyl-piperidino-, 2-Ethyl-piperidino-, 2,6-Dimethylpiperidino- oder Morpholinogruppe.

Die neuen Verbindungen werden wie folgt hergestellt: Durch Umsetzung eines 5,10-Dihydro-11H-dibenzo[b,e] [1,4]diazepin-11-ons der allgemeinen Formel II,

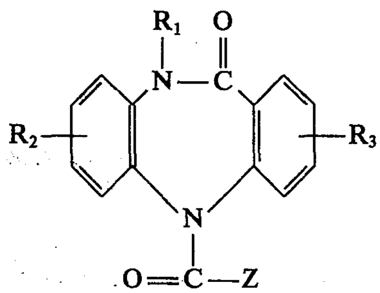

(II)

in der

$R_1$, $R_2$ und $R_3$ wie oben definiert sind und
Z die Gruppe $-CH_2-CH_2-Hal$ oder $-CH=CH_2$ bedeutet,

wobei Hal ein Halogenatom, insbesondere ein Chlor- oder Bromatom, darstellt, mit einem Amin der allgemeinen Formel III,

$$H-R_4 \qquad \text{(III)}$$

in der $R_4$ die obengenannten Gruppen bedeutet.

Die Umsetzung erfolgt, sofern Z die Gruppe $-CH_2-CH_2-Hal$ bedeutet, vorteilhaft in einem indifferenten Lösungsmittel, gegebenenfalls unter Zusatz eines säurebindenden Mittels, bei Temperaturen bis zum Siedepunkt des Reaktionsgemisches. Als Lösungsmittel werden vorzugsweise Alkohole, wie Ethanol, n-Propanol, Isopropanol, Ketone, wie Aceton, Ether wie Dioxan oder Tetrahydrofuran oder auch Dimethylformamid verwendet; es können aber auch aromatische Kohlenwasserstoffe, wie Benzol oder Toluol, eingesetzt werden. Es ist zweckmäßig, das Amin der allgemeinen Formel III in einem mindestens 2molaren Überschuß einzusetzen, um den freiwerdenden Halogenwasserstoff zu binden; man kann aber auch andere, halogenwasserstoffbindende Mittel, z. B. Alkalicarbonate, Alkalihydrogencarbonate oder tertiäre Amine, wie Triethylamin, Pyridin oder Dimethylanilin, einsetzen.

Die Umsetzung kann unter Abspaltung von Halogenwasserstoff über eine Verbindung der allgemeinen Formel II, in der sich anstelle der Gruppe $-CO-CH_2-CH_2-Hal$ die entsprechende Acryloylgruppe befindet, verlaufen. Es lagert sich dann das Amin der allgemeinen Formel III an diese

Acryloylgruppe an. Bei der Umsetzung einer Verbindung der allgemeinen Formel II, in der Z die Gruppe $-CH_2-CH_2-Hal$ bedeutet, entsteht also unter bestimmten Bedingungen zunächst eine Verbindung der allgemeinen Formel II, in der Z die Gruppe $-CH=CH_2$ darstellt, die in situ mit dem Amin der allgemeinen Formel III weiter reagiert.

Entsprechend lassen sich die Verbindungen der allgemeinen Formel I aber auch dadurch herstellen, daß man zuerst einer Verbindung der allgemeinen Formel II, in der Z die Gruppe $-CH_2-CH_2-Hal$ darstellt, und die in einem inerten Lösungsmittel gelöst ist, durch Erhitzen, vorzugsweise auf Rückflußtemperatur des Reaktionsgemisches, mittels eines halogenwasserstoffbindenden Mittels, den Halogenwasserstoff entzieht, die dabei entstehende Verbindung der allgemeinen Formel II, in der Z die Gruppe $-CH=CH_2$ bedeutet, isoliert und diese Verbindung anschließend in einem geeigneten Lösungsmittel mit einem Amin der allgemeinen Formel III bei Temperaturen bis zum Siedepunkt des Reaktionsgemisches umsetzt.

Als Lösungsmittel bei der Abspaltung von Halogenwasserstoff dienen bei dieser Reaktionsweise beispielsweise Alkohole wie Ethanol, höhersiedende Ether wie Dioxan oder Tetrahydrofuran, aber auch aromatische Kohlenwasserstoffe wie Benzol oder Toluol, als halogenwasserstoffabspaltende Mittel z. B. Alkalicarbonate, Alkalihydrogencarbonate oder tertiäre organische Amine wie Triethylamin, Pyridin oder Dimethylanilin. Die Umsetzung des so entstehenden Zwischenprodukts mit dem Amin der allgemeinen Formel III erfolgt in einem Lösungsmittel, beispielsweise in einem Alkohol, wie Ethanol, n-Propanol, Isopropanol oder in einem Keton wie Aceton oder in einem Ether wie Dioxan oder Tetrahydrofuran oder in einem aromatischen Kohlenwasserstoff, wie Benzol oder Toluol und bei Temperaturen bis zum Siedepunkt des Reaktionsgemisches.

Die Verbindungen der allgemeinen Formel I können durch Umsetzung mit anorganischen oder organischen Säuren nach bekannten Methoden in ihre physiologisch verträglichen Salze übergeführt werden. Als Säuren haben sich beispielsweise Salzsäure, Bromwasserstoffsäure, Schwefelsäure, Phosphorsäure, Weinsäure, Fumarsäure, Zitronensäure, Maleinsäure, Bernsteinsäure oder Oxalsäure als geeignet erwiesen.

Ein weiterer Gegenstand vorliegender Erfindung besteht in der Bereitstellung von Arzneimitteln, welche die Verbindungen der allgemeinen Formel I und/oder ihre physiologisch verträglichen Salze mit anorganischen oder organischen Säuren enthalten. Die Arzneimittel können in Form von Tabletten, Dragées, Ampullen Suppositorien oder Lösungen zum Einnehmen vorliegen. Die Einzeldosis beträgt im allgemeinen bei Erwachsenen bei peroraler Applikation 0,5 bis 50 mg, die bevorzugte Einzeldosis 1 bis 10 mg, die Tagesdosis kann dabei zwischen 1 und 100 mg, vorzugsweise 2 bis 20 mg, schwanken, entsprechend den individuellen Bedürfnissen des Patienten.

Die Ausgangsverbindungen der allgemeinen Formel II können wie folgt hergestellt werden:

Durch Umsetzung eines 5,10-Dihydro-11H-dibenzo[b,e] [1,4]diazepin-11-ons der allgemeinen Formel IV,

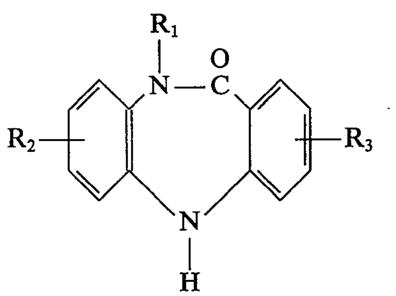

(IV)

in der

$R_1$, $R_2$ und $R_3$ wie oben definiert sind, mit einem Halogenpropionylhalogenid der allgemeinen Formel V oder einem Acrylsäurehalogenid der allgemeinen Formel VI

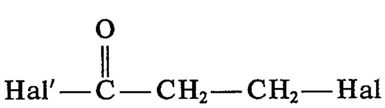

(V)

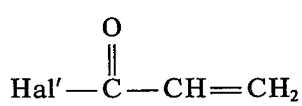

(VI)

in der

Hal und Hal', die gleich oder verschieden sein können, Halogenatome, wie Chlor, Brom oder Jod bedeuten. Im Falle der Umsetzung mit einer Verbindung der allgemeinen Formel V entsteht eine Verbindung der allgemeinen Formel II, in der Z die Gruppe $-CH_2-CH_2-Hal$ bedeutet; im Falle der Umsetzung mit einer Verbindung der allgemeinen Formel VI entsteht eine Verbindung der allgemeinen Formel II, in der Z die Gruppe $-CH=CH_2$ darstellt. Die Umsetzung erfolgt vorzugsweise in einem inerten Lösungsmittel, gegebenenfalls in Gegenwart eines halogenwasserstoffbindenden

Mittels, bei erhöhten Temperaturen, vorzugsweise bei der Siedetemperatur des Reaktionsgemisches. Als Lösungsmittel können aromatische Kohlenwasserstoffe, z. B. Benzol, Toluol, Xylol oder Ether, wie Diethylether, Dipropylether oder vorzugsweise cyclische Ether, wie Dioxan verwendet werden. Als halogenwasserstoffbindende Mittel eignen sich tertiäre organische Amine, wie Triethylamin, N,N-Dimethylanilin und Pyridin oder auch anorganische Basen, wie Alkalicarbonate oder Alkalihydrogencarbonate. Die Aufarbeitung des Reaktionsgemisches erfolgt in üblicher Weise, die Ausbeuten betragen bis zu 90% der Theorie. Die gebildeten Halogenpropionylverbindungen der allgemeinen Formel II sind meist gut kristallisierbare Substanzen, die auch ohne weiter Reinigung als Rohprodukte für die weitere Umsetzung verwendet werden können.

Es wurden beispielsweise durch Umsetzung der entsprechenden Verbindung der allgemeinen Formel IV mit Verbindungen der allgemeinen Formel V die folgenden Verbindungen hergestellt (z. B. mit 3-Chlorpropionylchlorid in Dioxan als Lösungsmittel):

a) 5-(3-Chlorpropionyl)-5,10-dihydro-11H-dibenzo[b,e] [1,4]diazepin-11-on, F. 217–218°C;
b) 5-(3-Chlorpropionyl)-5,10-dihydro-1-methyl-11H-dibenzo[b,e] [1,4]diazepin-11-on, F. 181–182°C (unter Zersetzung);
c) 2-Chlor-5-(3-chlorpropionyl)-5,10-dihydro-10-methyl-11H-dibenzo[b,e] [1,4]diazepin-11-on, F. 140–142°C;
d) 2-Chlor-5-(3-chlorpropionyl)-5,10-dihydro-11H-dibenzo[b,e] [1,4]diazepin-11-on, F. 169–171°C;
e) 5-(3-Chlorpropionyl)-10-ethyl-5,10-dihydro-11H-dibenzo[b,e] [1,4]diazepin-11-on, F. 133–134°C;
f) 8-Chlor-5-(3-chlorpropionyl)-5,10-dihydro-11H-dibenzo[b,e] [1,4]diazepin-11-on, F. 235–237°C.

Durch Umsetzung entsprechender Verbindungen der allgemeinen Formel IV mit Acrylsäurehalogeniden der allgemeinen Formel VI wurde die folgende Verbindung hergestellt:

g) 5-(Acryloyl)-5,10-dihydro-11H-dibenzo[b,e] [1,4]diazepin-11-on, F. 255–257°C (Zers.) (aus Ethanol).

Man kann aber auch die 5-(3-Halogenpropionyl)-5,10-dihydro-11H-dibenzo[b,e] [1,4]diazepin-11-one leicht, wie oben bereits angeführt, in die in 11-Stellung eine Acryloylgruppe enthaltenden Ausgangsverbindungen der allgemeinen Formel II, in der Z die Gruppe $-CH{=}CH_2$ bedeutet, überführen. So erhält man beispielsweise aus dem 5-(3-Chlorpropionyl)-5,10-dihydro-11H-dibenzo[b,e] [1,4]diazepin-11-on in Ethanol als Lösungsmittel nach halbstündigem Kochen bei Rückflußtemperatur in Gegenwart eines Überschusses an Triethylamin in guter Ausbeute das 5-(Acrylolyl)-5,10-dihydro-11H-dibenzo[b,e] [1,4]diazepin-11-on vom F. 255–257°C (Zers.) (aus Ethanol). Es ist nicht erforderlich, die Acryloylverbindungen aus dem Reaktionsgemisch zu isolieren, man kann sie im Reaktionsgemisch durch Zugabe des entsprechenden Amins der allgemeinen Formel III direkt zu dem gewünschten Endprodukt der allgemeinen Formel I weiter umsetzen.

Die Verbindungen der allgemeinen Formel IV sind literaturbekannt (vgl. z. B. Hunziker, Arzneimittelforschung 13, 324 [1963] und GB-A-1 236 112 [Thomae]).

Die neuen Verbindungen der allgemeinen Formel I und ihre Salze weisen wertvolle therapeutische Eigenschaften auf, sie wirken insbesondere ulkushemmend und sekretionshemmend und sind in diesen Eigenschaften, aber auch bezüglich des Fehlens bestimmter Nebenwirkungen, den chemisch ähnlichen Verbindungen der DE-C-1 795 176 überlegen.

Es wurden beispielsweise die folgenden Verbindungen

5,10-Dihydro-5-[3-(pyrrolidino)propionyl]-11H-dibenzo[b,e] [1,4]diazepin-11-on-hydrochlorid = A,
5,10-Dihydro-10-methyl-5-[3-(pyrrolidino)propionyl]-11H-dibenzo[b,e] [1,4]diazepin-11-on-hydrochlorid = B
und
5,10-Dihydro-5-[3-(piperidino)propionyl]-11H-dibenzo[b,e] [1,4]diazepin-11-on-hydrochlorid = C

im Hinblick auf ihre Magensekretionshemmwirkung, ihre Ulkushemmwirkung, ihre Mydriasiswirkung und die akute Toxizität untersucht und mit den folgenden vorbekannten Verbindungen verglichen:

5,10-Dihydro-5-pyrrolidinoacetyl-11H-dibenzo[b,e] [1,4]diazepin-11-on = D,
5,10-Dihydro-10-methyl-5-pyrrolidinoacetyl-11H-dibenzo[b,e] [1,4]diazepin-11-on-hydrochlorid = E
und
5,10-Dihydro-5-piperidinoacetyl-11H-dibenzo[b,e] [1,4]diazepin-11-on = F.

Die Hemmwirkung auf die Magensaftsekretion wurde nach der Methode von H. Shay et al., Gastroenterology 26, 906 (1954) an männlichen Crl : COBS (WI)BR Albino-Ratten von einem Gewichts

von 100 bis 125 g untersucht, wobei die Ratten vor der Untersuchung für 24 Stunden nüchern gehalten wurden. Unter Etheranaesthesie wurde der Bauch geöffnet und der Magenausgang zugebunden. Die zu applizierenden Substanzen wurden in einem konstanten Volumen von 0,2 ml/100 g Körpergewicht in das Duodenum unmittelbar nach der Ligatur des Pylorus injiziert, wobei jede Dosis an eine Gruppe von jeweils 10 Ratten verabreicht wurde. Kontrolltiere erhielten ein gleiches Volumen des Vehikels (0,2%ige Methylcelluloselösung). Nach 4 Stunden wurden die Tiere durch Ausrenken des Halswirbels getötet, es wurde der Magen entnommen, entlang der großen Kurvatur aufgeschnitten und der Mageninhalt gesammelt. Es wurde der gesamte Gehalt der Magenflüssigkeit an Salzsäure durch Titration mit N/10 Natriumhydroxid-Lösung bestimmt. Die Ergebnisse wurden als die prozentualen Verhältnisse aus den Daten, die von den behandelten und unbehandelten Tieren erhalten wurden, bestimmt und die $ED_{50}$-Werte wurden durch lineare Regressionsanalyse der Probits nach D. J. Finney, Probit Analysis, Cambridge Unitversity Press, 1971, berechnet.

Die Hemmwirkung auf die Bildung von Magenulzera wurde an weiblichen Crl : COBS-CD(SD)BR Ratten von einem Körpergewicht von 130 bis 160 g nach der Methode von P. A. Brown, European J. Pharmacol. 51, 275 (1978) untersucht. Die zu applizierenden Substanzen wurden in einer 0,2%igen Methylcellulose-Lösung suspendiert und den Tieren, die vorher für 24 Stunden nüchtern gehalten wurden, per Schlundsonde verabreicht. 30 Minuten nach der Verabreichung der zu untersuchenden Verbindungen erhielten die Ratten jeweils 8 mg/kg Körpergewicht Acetylsalicylsäure peroral, 30 Minuten später wurden die Tiere bei Zimmertemperatur in ein engmaschiges Drahtnetz eingesperrt. Nach einer Stunde Aufenthalt in diesem Drahtnetz wurden die Tiere durch Ausrenken des Halswirbels getötet; es wurde der Magen entnommen, entlang der kleinen Kurvatur aufgeschnitten und die Innenseite nach außen gekehrt auf einem Reagenzglas aufgezogen, wobei die Anwesenheit von Magenulzera registriert wurde. Die Wirkung der Verbindungen wurde dadurch festgestellt, daß die Anzahl der Mägen, die frei von Ulzera waren, bei den behandelten Ratten gezählt wurde. Nach der Methode von J. T. Litchfield und F. Wilcoxon, J. Pharmac. Exp. Therap. 96, 99 (1949) wurde dann die $ED_{50}$ berechnet.

Die Wirkung auf die Größe der Pupille wurde an männlichen Crl : COBS-CD(SD)BR Ratten von einem Körpergewicht von 100 bis 130 g untersucht; verwendet wurde ein Nachet-Binokular-Mikroskop mit einer graduierten Linse und einer 10fachen Vergrößerung.

Die Verbindungen wurden in einer 0,2%igen Methylcellulose-Lösung suspendiert und mittels einer Schlundsonde verabreicht, pro Dosis wurden Gruppen von 5 Ratten verwendet. Kontrolltiere erhielten dasselbe Volumen (1 ml/100 g Körpergewicht) des Vehikels. Der Durchmesser der Pupille wurde vorher und 30, 60, 90 und 120 Minuten nach der Behandlung gemessen; die Wirkung wurde in Prozent, bezogen auf den mit Atropin erhaltenen maximalen Durchmesser, ausgedrückt. Es wurde dann die $ED_{50}$ durch eine lineare Regressionsanalyse der Probits nach D. J. Finney bestimmt.

Die akute Toxizität wurde an männlichen Crl : COBS-CD-I(ICR) Mäusen mit einem Körpergewicht von 22 bis 24 g, die vorher für 15 Stunden nüchtern gehalten wurden, bestimmt. Es wurden verschiedene Dosen jeder Verbindung durch Schlundsonde an Gruppen von jeweils 5 bis 10 Tieren verabreicht, und die $LD_{50}$-Werte nach der Methode von D. J. Finney errechnet.

Die folgende Tabelle enthält die gefundenen Werte:

| Substanz | Hemmung der Magensaftsekretion (Säurefreisetzung) (Ratte) | Ulkushemmung (Ratte) | Mydriasis (Ratte) | Akute Toxizität (Maus) |
|---|---|---|---|---|
| | $ED_{50}$ mg/kg i.d. | $ED_{50}$ mg/kg p.o. | $ED_{50}$ mg/kg p.o. | $LD_{50}$ mg/kg p.o. |
| A | 0,20 | 1,27 | 3,61 | 609 |
| B | 0,73 | 2,05 | | 574 |
| C | 1,60 | 2,10 | 3,84 | 813 |
| D | 8,15 | 10,20 | 9,91 | 860 |
| E | 18,05 | 8,40 | 9,84 | 816 |
| F | 6,17 | 10,40 | 5,82 | 600 |

Die Substanzen A, B und C zeigen eine signifikant stärkere Hemmwirkung auf die

Magensaftsekretion bzw. Freisetzung von Salzsäure und dementsprechend auf die Bildung von Ulzera als die Substanzen D, E und F. Darüber hinaus ist für die Substanzen A und C das Verhältnis der $ED_{50}$ der Ulkushemmwirkung zu der $ED_{50}$ der mydriatischen Wirkung kleiner als 1, d. h. diese Substanzen zeigen bei ihrer therapeutisch richtigen Dosierung noch keine mydriatische Wirkung.

Die nachfolgenden Beispiele sollen die Erfindung näher erläutern:

## Beispiel 1

### 5,10-Dihydro-5-[3-(pyrrolidino)propionyl]-11H-dibenzo[b,e] [1,4]-diazepin-11-on-hydrochlorid

60,0 g (0,2 Mol) 5-(3-Chlorpropionyl)-5,10-dihydro-11H-dibenzo[b,e] [1,4]diazepin-11-on und 35,5 g (0,5 Mol) Pyrrolidin in 600 ml Isopropanol wurden 45 Minuten unter Rückfluß erhitzt. Nach Zugabe von Aktivkohle wurde filtriert und das Filtrat im Vakuum eingedampft. Der Rückstand wurde mit Wasser verrührt, die kristalline Substanz abgesaugt und aus Isopropanol umkristallisiert. Man erhielt 55 g 5,10-Dihydro-5-[3-(pyrrolidino)propionyl]-11H-dibenzo[b,e] [1,4]diazepin-11-on vom F. 171 – 173° C.

Diese Base wurde unter Erwärmen in 200 ml n-Propanol gelöst und mit der berechneten Menge konzentrierter Salzsäure versetzt. Man erhielt 55,5 g Hydrochlorid vom F. 241 – 243° C. Ausbeute: 75% der Theorie.

## Beispiel 2

### 5,10-Dihydro-10-methyl-5-[3-(pyrrolidino)propionyl]-11H-dibenzo[b,e] [1,4]-diazepin-11-on-hydrochlorid

4,5 g (0,015 Mol) 5-(3-Chlorpropionyl)-5,10-dihydro-10-methyl-11H-dibenzo[b,e] [1,4]diazepin-11-on und 2,6 g (0,0375 Mol) Pyrrolidin wurden in 45 ml Isopropanol 2 Stunden unter Rückfluß erhitzt und wie in Beispiel 1 beschrieben aufgearbeitet. Man erhielt 3,9 g des Hydrochlorids vom F. 235 – 237° C (umkristallisiert aus Isopropanol). Ausbeute: 67% der Theorie.

## Beispiel 3

### 2-Chlor-5,10-dihydro-5-[3-(pyrrolidino)propionyl]-11H-dibenzo[b,e] [1,4]diazepin-11-on

6,7 g (0,02 Mol) 2-Chlor-5-(3-chlorpropionyl)-5,10-dihydro-11H-dibenzo[b,e] [1,4]diazepin-11-on und 7,1 g (0,1 Mol) Pyrrolidin wurden in 50 ml Isopropanol 2 Stunden unter Rückfluß erhitzt. Nach Zugabe von Aktivkohle wurde filtriert und das Filtrat im Vakuum eingedampft. Der kristalline Rückstand wurde aus 50%igem wäßrigem Isopropanol umkristallisiert. Man erhielt 4,3 g der Verbindung vom F. 167 – 169° C. Ausbeute: 58% der Theorie.

## Beispiel 4

### 2-Chlor-5,10-dihydro-10-methyl-5-[3-(pyrrolidino)propionyl]-11H-dibenzo[b,e] [1,4]diazepin-11-on

3,5 g (0,01 Mol) 2-Chlor-5-(3-chlorpropionyl)-5,10-dihydro-10-methyl-11H-dibenzo[b,e]— [1,4]diazepin-11-on und 3,5 g (0,05 Mol) Pyrrolidin wurden in 50 ml Dioxan 4 Stunden unter Rückfluß erhitzt. Nach Zugabe von Aktivkohle wurde filtriert und das Filtrat im Vakuum eingedampft. Der Rückstand wurde in Chloroform gelöst, diese Lösung wurde mit Wasser ausgeschüttelt und die organische Phase im Vakuum eingedampft. Der ölige Rückstand wurde durch Zugabe von Ether kristallin. Nach dem Umkristallisieren aus Cyclohexan erhielt man 2,6 g Kristalle vom F. 118 – 120° C. Ausbeute: 67% der Theorie.

## Beispiel 5

### 5,10-Dihydro-5-[3-(piperidino)propionyl]-11H-dibenzo[b,e] [1,4]-diazepin-11-on-hydrochlorid

60,0 g (0,2 Mol) 5-(3-Chlorpropionyl)-5,10-dihydro-11H-dibenzo[b,e] [1,4]diazepin-11-on und 42,5 g

(0,5 Mol) Piperidin wurden in 600 ml Isopropanol nach der im Beispiel 1 beschriebenen Weise umgesetzt und aufgearbeitet. Man erhielt 53 g des Hydrochlorids (umkristallisiert aus n-Propanol) vom F. 250 – 252° C. Ausbeute: 69% der Theorie. (Schmelzpunkt der Base: F. 170 – 172° C, umkristallisiert aus Isopropanol.)

Beispiel 6

5,10-Dihydro-10-methyl-5-[3-(piperidino)propionyl]-11H-dibenzo[b,e] [1,4]-diazepin-11-on-hydrochlorid

6,3 g (0,02 Mol) 5-(3-Chlorpropionyl)-5,10-dihydro-10-methyl-11H-dibenzo[b,e] [1,4]diazepin-11-on und 4,25 g (0,05 Mol) Piperidin wurden in 100 ml Dimethylformamid gelöst und 60 Stunden bei Raumtemperatur stehengelassen. Das ausgefallene Piperidinhydrochlorid wurde abfiltriert, das Filtrat im Vakuum eingedampft, der Rückstand in Ethanol gelöst, mit Salzsäure auf pH 2 eingestellt und das Lösungsmittel im Vakuum abdestilliert. Der Rückstand wurde aus Isopropanol/Essigsäureethylester = 1 : 1 umkristallisiert. Man erhielt 4,4 g des Hydrochlorids vom F. 248 – 249° C. Ausbeute: 55% der Theorie.

Beispiel 7

2-Chlor-5,10-dihydro-5-[3-(piperidino)propionyl]-11H-dibenzo[b,e] [1,4]diazepin-11-on

5,0 g (0,015 Mol) 2-Chlor-5-(3-chlorpropionyl)-5,10-dihydro-11H-dibenzo[b,e] [1,4]diazepin-11-on und 4,3 g (0,05 Mol) Piperidin wurden in 100 ml Dioxan 1 Stunde lang unter Rückfluß erhitzt. Das Reaktionsgemisch wurde im Vakuum eingedampft, der Rückstand in verdünnter Essigsäure gelöst und nach Zusatz von Aktivkohle filtriert. Das Filtrat wurde mit Ammoniak alkalisch gemacht und mit Chloroform ausgeschüttelt. Die Chloroformlösung wurde im Vakuum eingedampft, der Rückstand aus Isopropanol kristallisiert und aus Methanol umkristallisiert. Man erhielt 2,9 g Substanz vom F. 170 – 172° C. Ausbeute: 50% der Theorie.

Beispiel 8

5,10-Dihydro-5-[3-(2-methylpiperidino)propionyl]-11H-dibenzo[b,e] [1,4]-diazepin-11-on-hydrogenfumarat

9,0 g (0,03 Mol) 5-(3-Chlorpropionyl)-5,10-dihydro-11H-dibenzo[b,e] [1,4]diazepin-11-on und 7,4 g (0,075 Mol) 2-Methylpiperidin wurden in 200 ml Isopropanol 4 Stunden unter Rückfluß erhitzt. Das Reaktionsgemisch wurde im Vakuum eingedampft und der Rückstand über eine Kieselgelsäule (Fließmittel: Chloroform + Isooctan + Methanol + Ammoniak = 5 + 2 + 3 + 0,3) gereinigt. Die erhaltene Base (F. 156 – 157° C, aus Essigester umkristallisiert) wurde unter Erwärmen in Essigsäureethylester gelöst, mit Fumarsäure versetzt und im Vakuum eingedampft. Der Rückstand wurde aus Essigsäureethylester/Methanol umkristallisiert. Man erhielt 6,0 g des Hydrogenfumarats vom F. 140 – 142° C. Ausbeute: 42% der Theorie.

Beispiel 9

5,10-Dihydro-10-methyl-5-[3-(2-methylpiperidino)propionyl]-11H-dibenzo[b,e] [1,4]-diazepin-11-on-hydrogensulfat

6,3 g (0,02 Mol) 5-(3-Chlorpropionyl)-5,10-dihydro-10-methyl-11H-dibenzo[b,e] [1,4]diazepin-11-on und 5 g (0,05 Mol) 2-Methylpiperidin wurden in 200 ml Isopropanol 2 Stunden unter Rückfluß erhitzt. Das Reaktionsgemisch wurde im Vakuum eingedampft, der Rückstand in Methylenchlorid gelöst und mit Wasser gewaschen. Die organische Phase wurde im Vakuum eingedampft, in Ethanol gelöst und mit 4,5 g 30%iger Schwefelsäure versetzt. Der Alkohol wurde teilweise abdestilliert und durch Zugabe von Essigsäureethylester das Hydrogensulfat zur Kristallisation gebracht. Man erhielt 3,8 g vom F. 215 – 217° C. Ausbeute: 40% der Theorie.

Beispiel 10

5,10-Dihydro-10-ethyl-5-[3-(2-methylpiperidino)propionyl]-11H-dibenzo[b,e] [1,4]-diazepin-11-on-hydrochlorid

6,6 g (0,02 Mol) 5-(3-Chlorpropionyl)-5,10-dihydro-10-ethyl-11H-dibenzo[b,e] [1,4]diazepin-11-on (F. 133–134°C) und 5 g (0,05 Mol) 2-Methylpiperidin wurden in 100 ml Isopropanol 2 Stunden unter Rückfluß erhitzt. Das Reaktionsgemisch wurde im Vakuum eingedampft, in Methylenchlorid gelöst und mit Wasser gewaschen. Nach Abdampfen des Lösungsmittels wurde der Rückstand in heißem Aceton gelöst und die berechnete Menge Salzsäure zugegeben. Es kristallisierten 4,3 g des Hydrochlorids vom F. 243–244° C aus. Ausbeute: 50% der Theorie.

Beispiel 11

2-Chlor-5,10-dihydro-5-[3-(2-methylpiperidino)propionyl]-11H-dibenzo[b,e] [1,4]diazepin-11-on

5,0 g (0,015 Mol) 2-Chlor-5-(3-chlorpropionyl)-5,10-dihydro-11H-dibenzo[b,e] [1,4]diazepin-11-on und 5 g (0,05 Mol) 2-Methylpiperidin wurden in 100 ml Dioxan 2 Stunden lang unter Rückfluß erhitzt und wie in Beispiel 7 beschrieben, aufgearbeitet. Man erhielt 3,3 g Substanz vom F. 199–210° C (nach Umkristallisation aus Isopropanol), Ausbeute: 54% der Theorie.

Beispiel 12

8-Chlor-5,10-dihydro-5-[3-(2-methylpiperidino)propionyl]-11H-dibenzo[b,e] [1,4]diazepin-11-on

11,5 g (0,034 Mol) 8-Chlor-5-(3-chlorpropionyl)-5,10-dihydro-11H-dibenzo[b,e] [1,4]diazepin-11-on (F. 235–237° C) und 16,2 g (0,16 Mol) 2-Methylpiperidin wurden in 100 ml Isopropanol 1 Stunde lang unter Rückfluß erhitzt. Das Reaktionsgemisch wurde im Vakuum eingedampft, der Rückstand in Chloroform gelöst, mit Natronlauge gewaschen und das organische Lösungsmittel im Vakuum abdestilliert. Der Rückstand wurde aus Xylol umkristallisiert. Man erhielt 12,0 g Substanz vom F. 201–202° C (unter Zersetzung). Ausbeute: 88% der Theorie.

Beispiel 13

5,10-Dihydro-5-[3-(2-ethylpiperidino)propionyl]-11H-dibenzo[b,e] [1,4]diazepin-11-on

6,0 g (0,02 Mol) 5-(3-Chlorpropionyl)-5,10-dihydro-11H-dibenzo[b,e] [1,4]diazepin-11-on und 8,5 g (0,075 Mol) 2-Ethylpiperidin wurden in 100 ml Dimethylformamid bei Raumtemperatur 18 Stunden lang gerührt. Das Lösungsmittel wurde im Vakuum abdestilliert, der Rückstand wurde über eine Kieselgelsäule (Fließmittel: Ethanol) gereinigt. Die erhaltene Base wurde aus Essigsäureethylester/Ether umkristallisiert. Man erhielt 3,6 g Substanz vom F. 147–148° C. Ausbeute: 48% der Theorie.

Beispiel 14

5,10-Dihydro-5-[3-(2-ethylpiperidino)propionyl]-10-methyl-11H-dibenzo[b,e] [1,4]diazepin-11-on-hydrochlorid

8,5 g (0,027 Mol) 5-(3-Chlorpropionyl)-5,10-dihydro-10-methyl-11H-dibenzo[b,e] [1,4]diazepin-11-on und 8,5 g (0,075 Mol) 2-Ethylpiperidin wurden in 85 ml n-Propanol 3 Stunden lang unter Rückfluß erhitzt. Die Aufarbeitung erfolgte wie im Beispiel 10 beschrieben. Nach dem Umkristallisieren aus einem Gemisch aus gleichen Teilen Dioxan und Aceton erhielt man 6,8 g des Hydrochlorids vom F. 209–211° C. Ausbeute: 58% der Theorie.

## Beispiel 15

### 5,10-Dihydro-5-[3-(2,6-dimethylpiperidino)propionyl]-10-methyl-11H-dibenzo[b,e] [1,4]diazepin-11-on

6,3 g (0,02 Mol) 5-(3-Chlorpropionyl)-5,10-dihydro-10-methyl-11H-dibenzo[b,e] [1,4]diazepin-11-on und 8,4 g (0,075 Mol) 2,6-Dimethylpiperidin wurden in 100 ml Isopropanol 6 Stunden unter Rückfluß erhitzt. Die Aufarbeitung erfolgte wie im Beispiel 13 beschrieben. Nach dem Umkristallisieren aus Essigsäureethylester unter Zusatz von wenig Ether erhielt man 4,2 g Substanz vom F. 143—145° C. Ausbeute: 54% der Theorie.

## Beispiel 16

### 5,10-Dihydro-5-[3-(morpholino)propionyl]-11H-dibenzo[b,e] [1,4]diazepin-11-on

6,0 g (0,02 Mol) 5-(3-Chlorpropionyl)-5,10-dihydro-11H-dibenzo[b,e] [1,4]diazepin-11-on und 10 g (0,11 Mol) Morpholin wurden in 200 ml Isopropanol 2 Stunden lang unter Rückfluß erhitzt, das Lösungsmittel abdestilliert, der Rückstand in Methylenchlorid gelöst und die Lösung mit Wasser gewaschen. Nach dem Abdestillieren des Lösungsmittels wurde der Rückstand aus Isopropanol umkristallisiert. Man erhielt 3,9 g Substanz vom F. 183—184° C. Ausbeute: 56% der Theorie.

## Beispiel 17

### 5,10-Dihydro-10-methyl-5-[3-(morpholino)propionyl]-11H-dibenzo[b,e] [1,4]-diazepin-11-on-hydrochlorid

6,3 g (0,02 Mol) 5-(3-Chlorpropionyl)-5,10-dihydro-10-methyl-11H-dibenzo[b,e] [1,4]diazepin-11-on und 10 g (0,11 Mol) Morpholin wurden in 200 ml Isopropanol 3 Stunden lang unter Rückfluß erhitzt. Nach dem Abdestillieren des Lösungsmittels wurde der Rückstand in Methylenchlorid gelöst, mit Wasser gewaschen und die organische Lösung im Vakuum eingedampft. Der Rückstand wurde in Ethanol gelöst und mit Salzsäure auf pH 4 gebracht. Das auskristallisierte Hydrochlorid wurde aus Isopropanol umkristallisiert. Man erhielt 3,9 g Substanz vom F. 256—257° C (Zersetzung). Ausbeute: 49% der Theorie.

## Beispiel 18

### 2-Chlor-5,10-dihydro-10-methyl-5-[3-(morpholino)propionyl]-11H-dibenzo[b,e] [1,4]diazepin-11-on

3,5 g (0,01 Mol) 2-Chlor-5-(3-chlorpropionyl)-5,10-dihydro-10-methyl-11H-dibenzo[b,e] [1,4]diazepin-11-on und 4,4 g (0,05 Mol) Morpholin wurden in 50 ml Dioxan 4 Stunden unter Rückfluß erhitzt. Die Aufarbeitung erfolgte wie im Beispiel 12 beschrieben. Nach dem Umkristallisieren aus Isopropanol unter Zusatz von wenig Ether erhielt man 2,5 g Substanz vom F. 138—140° C. Ausbeute: 62% der Theorie.

## Beispiel 19

### 5,10-Dihydro-5-[3-(pyrrolidino)propionyl]-11H-dibenzo[b,e] [1,4]diazepin-11-on

Herstellung der Ausgangsverbindung:
5-Acryloyl-5,10-dihydro-11H-dibenzo[b,e] [1,4]diazepin-11-on

a) 5,0 g (0,017 Mol) 5-(3-Chlorpropionyl)-5,10-dihydro-11H-dibenzo[b,e] [1,4]diazepin-11-on und 2,0 g (0,02 Mol) Triethylamin in 250 ml Ethanol wurden 30 Minuten unter Rückfluß erhitzt und anschließend im Vakuum eingedampft. Der Rückstand wurde mit Wasser verrührt. Nach dem Absaugen und Trocknen erhielt man 5-Acryloyl-5,10-dihydro-11H-dibenzo[b,e] [1,4]diazepin-11-on in quantitativer Ausbeute. Nach dem Umkristallisieren aus Ethanol: F. 255—257° C (Zers.)

b) 5,26 g (0,025 Mol) 5,10-Dihydro-11H-dibenzo[b,e] [1,4]diazepin-11-on und 2,7 g (0,03 Mol) Acrylsäurechlorid in 200 ml Toluol wurden unter Rühren 3 Stunden unter Rückfluß gekocht. Anschließend wurde im Vakuum zur Trockene eingeengt und der Rückstand mit Natriumbicarbonatlösung verrührt. Nach dem Absaugen, Waschen mit Wasser und Trocknen erhielt man in

quantitativer Ausbeute 5-Acryloyl-5,10-dihydro-11H-dibenzo[b,e] [1,4]diazepin-11-on. Nach dem Umkristallisieren aus Ethanol: F. 255–257°C (Zers.).

Herstellung des Endprodukts:
5,10-Dihydro-5-[3-(pyrrolidino)propionyl]-11H-dibenzo[b,e] [1,4]diazepin-11-on

5,3 g (0,02 Mol) 5-Acryloyl-5,10-dihydro-11H-dibenzo[b,e] [1,4]diazepin-11-on und 1,6 g (0,022 Mol) Pyrrolidin wurden in 200 ml Ethanol 30 Minuten unter Rückfluß und Rühren erhitzt. Nach Zugabe von Aktivkohle wurde heiß filtriert und das Filtrat im Vakuum eingedampft. Der Rückstand wurde aus wenig Essigsäureethylester umkristallisiert. Man erhielt 5,7 g der Verbindung vom Fp.: 171–173°C. Ausbeute: 85% der Theorie.

Beispiel 20

5,10-Dihydro-5-[3-(pyrrolidino)propionyl]-11H-dibenzo[b,e] [1,4]diazepin-11-on

6,3 g (0,03 Mol) 5,10-Dihydro-11H-dibenzo[b,e] [1,4]diazepin-11-on und 3,3 g (0,036 Mol) Acrylsäurechlorid wurden in 200 ml Toluol unter Rühren 3 Stunden unter Rückfluß erhitzt. Das Reaktionsgemisch wurde im Vakuum eingeengt. Der trockene Rückstand wurde mit 200 ml Ethanol versetzt und in die kochende Lösung wurden unter Rühren 2,5 g (0,035 Mol) Pyrrolidin zugetropft. Anschließend wurde noch 20 Minuten erhitzt, im Vakuum auf etwa 1/10 des Volumens eingeengt und durch Filtration über Aktivkohle/Kieselgel entfärbt. Das Rohprodukt wurde aus wenig Essigsäureethylester umkristallisiert. Man erhielt 8,8 g Substanz vom Fp.: 171–173°C. Ausbeute: 87% der Theorie.

Die nachfolgenden Beispiele sollen die Herstellung einiger pharmazeutischer Zubereitungsformen verdeutlichen:

Beispiel I

Tabletten mit 5 mg 5,10-Dihydro-5-[3-(pyrrolidino)propionyl]-11H-dibenzo[b,e] [1,4]diazepin-11-on-hydrochlorid

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 5 mg 5,10-Dihydro-5-[3-(pyrrolidino)propionyl]-11H-dibenzo[b,e] [1,4]diazepin-11-on-hydrochlorid

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 300 mg

## Beispiel III

Ampullen mit 1 mg 5,10-Dihydro-5-[3-(pyrrolidino)propionyl]-11H-dibenzo[b,e] [1,4]diazepin-11-on-hydrochlorid

Zusammensetzung:

1 Ampulle enthält:

| | | |
|---|---|---|
| Wirkstoff | | 1,0 mg |
| Natriumchlorid | | 8,0 mg |
| Dest. Wasser | ad | 1 ml |

### Herstellungsverfahren

Die Wirksubstanz und Natriumchlorid werden in dest. Wasser gelöst und anschließend auf das gegebene Volumen aufgefüllt. Die Lösung wird sterilfiltriert und in 1-ml-Ampullen abgefüllt.

Sterilisation: 20 Minuten bei 120° C.

## Beispiel IV

Suppositorien mit 5 mg 5,10-Dihydro-5-[3-(pyrrolidino)propionyl]-11H-dibenzo[b,e] [1,4]diazepin-11-on-hydrochlorid

Zusammensetzung:

1 Zäpfchen enthält:

| | |
|---|---|
| Wirkstoff | 5,0 mg |
| Zäpfchenmasse (z. B. Witepsol W 45®) | 1 695,0 mg |
| | 1 700,0 mg |

### Herstellungsverfahren

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.

Zäpfchengewicht: 1,7 g

## Beispiel V

Tropfen mit 5,10-Dihydro-5-[3-(pyrrolidino)propionyl]-11H-dibenzo[b,e] [1,4]diazepin-11-on-hydrochlorid, enthaltend 0,5 g Wirkstoff/100 ml Lösung

Zusammensetzung:

100 ml Tropflösung enthalten:

| | | |
|---|---|---|
| p-Oxybenzoesäuremethylester | | 0,035 g |
| p-Oxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

### Herstellungsverfahren

Die Wirksubstanz und Natriumcyclamat werden in ca. 70 ml Wasser gelöst und Glycerin zugefügt. Man löst die p-Oxybenzoesäureester, Anisöl sowie Menthol in Ethanol und fügt diese Lösung unter

Rühren der wäßrigen Lösung zu. Abschließend wird mit Wasser auf 100 ml aufgefüllt und schwebeteilchenfrei filtriert.

**Patentansprüche für die Vertragsstaaten: BE, CH, LI, DE, FR, IT, LU, NL, SE**

1. In 5-Stellung substituierte 5,10-Dihydro-11H-dibenzo[b,e] [1,4]diazepin-11-one der allgemeinen Formel I,

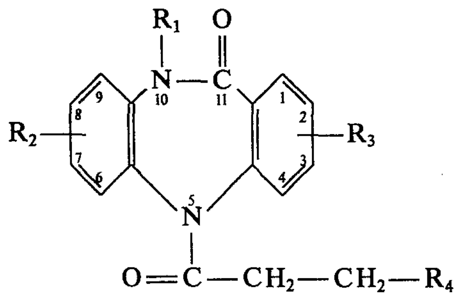

(I)

in der

$R_1$ ein Wasserstoffatom, die Methyl- oder Ethylgruppe,
$R_2$ ein Wasserstoff- oder Chloratom,
$R_3$ ein Wasserstoff- oder Chloratom und
$R_4$ die Pyrrolidino-, Piperidino-, 2-Methylpiperidino-, 2-Ethyl-piperidino-, 2,6-Dimethylpiperidino- oder Morpholinogruppe

bedeuten und deren physiologisch verträgliche Salze mit anorganischen oder organischen Säuren.

2. In 5-Stellung substituierte 5,10-Dihydro-11H-dibenzo[b,e] [1,4]diazepin-11-one der allgemeinen Formel I gemäß Anspruch 1, dadurch gekennzeichnet, daß

$R_1$ ein Wasserstoffatom oder die Methylgruppe,
$R_2$ und $R_3$ Wasserstoffatome und
$R_4$ die Pyrrolidino- oder Piperidinogruppe

bedeuten und deren physiologisch verträgliche Salze mit anorganischen oder organischen Säuren.

3. 5,10-Dihydro-5-[3-(pyrrolidino)propionyl]-11H-dibenzo[b,e] [1,4]diazepin-11-on und dessen physiologisch verträgliche Salze mit anorganischen oder organischen Säuren.

4. 5,10-Dihydro-10-methyl-5-[3-(pyrrolidino)propionyl]-11H-dibenzo[b,e] [1,4]diazepin-11-on und dessen physiologisch verträgliche Salze mit anorganischen oder organischen Säuren.

5. 5,10-Dihydro-5-[3-(piperidino)propionyl]-11H-dibenzo[b,e] [1,4]diazepin-11-on und dessen physiologisch verträgliche Salze mit anorganischen oder organischen Säuren.

6. Arzneimittel, enthaltend eine oder mehrere Verbindungen der allgemeinen Formel I, gemäß Anspruch 1 neben den üblichen Träger- und/oder Hilfsstoffen.

7. Verwendung einer oder mehrerer Verbindungen der allgemeinen Formel I gemäß Anspruch 1 zur Herstellung von Arzneimitteln auf nichtchemischem Wege.

8. Verfahren zur Herstellung von in 5-Stellung substituierten 5,10-Dihydro-11H-dibenzo[b,e] [1,4]diazepin-11-onen der allgemeinen Formel I,

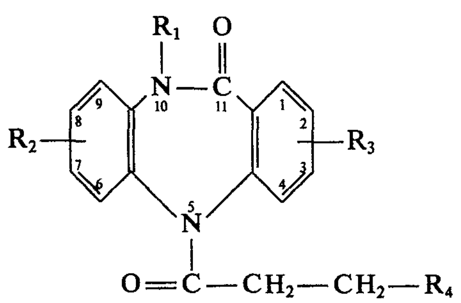

(I)

in der

$R_1$ ein Wasserstoffatom, die Methyl- oder Ethylgruppe,
$R_2$ ein Wasserstoff- oder Chloratom,

$R_3$ ein Wasserstoff- oder Chloratom und
$R_4$ die Pyrrolidino-, Piperidino-, 2-Methyl-piperidino-, 2-Ethyl-piperidino-, 2,6-Dimethylpiperidino- oder Morpholinogruppe

bedeuten und von deren Salzen mit anorganischen oder organischen Säuren, dadurch gekennzeichnet, daß ein 5,10-Dihydro-11H-dibenzo[b,e] [1,4]diazepin-11-on der allgemeinen Formel II,

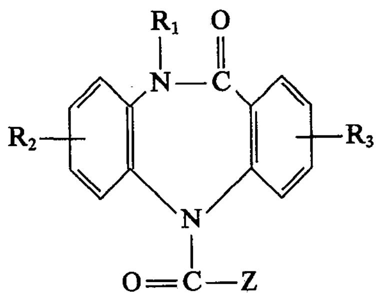

(II)

in der $R_1$, $R_2$ und $R_3$ wie oben definiert sind und Z die Gruppe $-CH_2-CH_2-Hal$ oder $-CH=CH_2$ bedeutet, wobei Hal ein Halogenatom darstellt, mit einem Amin der allgemeinen Formel III,

$$H-R_4 \qquad (III)$$

in der $R_4$ die obengenannten Bedeutungen besitzt, umgesetzt wird und, gewünschtenfalls, anschließend eine so erhaltene Verbindung der allgemeinen Formel I in ihre Salze mittels anorganischer oder organischer Säuren übergeführt wird.

9. Verfahren gemäß Anspruch 8, dadurch gekennzeichnet, daß eine Verbindung der allgemeinen Formel II, in der Z die Gruppe $-CH_2-CH_2-Hal$ bedeutet, wobei Hal ein Halogenatom, vorzugsweise ein Chlor- oder Bromatom, darstellt, in einem indifferenten organischen Lösungsmittel bei Temperaturen bis zum Siedepunkt des Reaktionsgemisches, mit entweder einem mindestens 2molaren Überschuß eines Amins der allgemeinen Formel III oder mit einem Amin der allgemeinen Formel III in Anwesenheit eines halogenwasserstoffbindenden Mittels umgesetzt wird.

10. Verfahren gemäß Anspruch 8, dadurch gekennzeichnet, daß eine Verbindung der allgemeinen Formel II, in der Z die Gruppe $-CH=CH_2$ bedeutet, in einem organischen Lösungsmittel, bei Temperaturen bis zum Siedepunkt des Reaktionsgemisches, mit einem Amin der allgemeinen Formel III umgesetzt wird.

**Patentansprüche für den Vertragsstaat: AT**

1. Verfahren zur Herstellung von in 5-Stellung substituierten 5,10-Dihydro-11H-dibenzo[b,e] [1,4]diazepin-11-onen der allgemeinen Formel I,

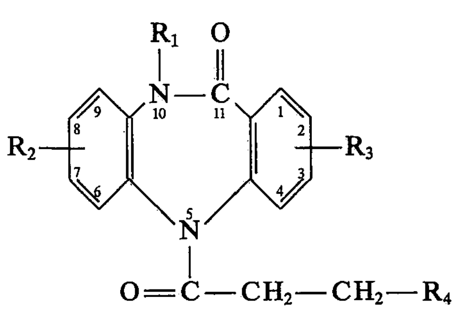

(I)

in der

$R_1$ ein Wasserstoffatom, die Methyl- oder Ethylgruppe,
$R_2$ ein Wasserstoff- oder Chloratom,
$R_3$ ein Wasserstoff- oder Chloratom und
$R_4$ die Pyrrolidino-, Piperidino-, 2-Methyl-piperidino-, 2-Ethyl-piperidino-, 2,6-Dimethylpiperidino- oder Morpholinogruppe

bedeuten und von deren Salzen mit anorganischen oder organischen Säuren, dadurch gekennzeichnet, daß ein 5,10-Dihydro-11H-dibenzo[b,e] [1,4]diazepin-11-on der allgemeinen Formel II,

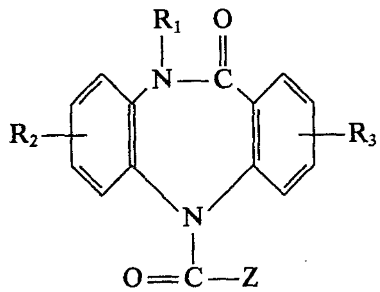

(II)

in der $R_1$, $R_2$ und $R_3$ wie oben definiert sind und Z die Gruppe $-CH_2-CH_2-Hal$ oder $-CH=CH_2$ bedeutet, wobei Hal ein Halogenatom darstellt, mit einem Amin der allgemeinen Formel III,

$$H-R_4 \qquad \text{(III)}$$

in der $R_4$ die obengenannten Bedeutungen besitzt, umgesetzt wird und, gewünschtenfalls, anschließend eine so erhaltene Verbindung der allgemeinen Formel I in ihre Salze mittels anorganischer oder organischer Säuren übergeführt wird.

2. Verfahren gemäß Anspruch 1, dadurch gekennzeichnet, daß eine Verbindung der allgemeinen Formel II, in der Z die Gruppe $-CH_2-CH_2-Hal$ bedeutet, wobei Hal ein Halogenatom, vorzugsweise ein Chlor- oder Bromatom, darstellt, in einem indifferenten organischen Lösungsmittel bei Temperaturen bis zum Siedepunkt des Reaktionsgemisches, mit entweder einem mindestens 2molaren Überschuß eines Amins der allgemeinen Formel III oder mit einem Amin der allgemeinen Formel III in Anwesenheit eines halogenwasserstoffbindenden Mittels umgesetzt wird.

3. Verfahren gemäß Anspruch 1, dadurch gekennzeichnet, daß eine Verbindung der allgemeinen Formel II, in der Z die Gruppe $-CH=CH_2$ bedeutet, in einem organischen Lösungsmittel, bei Temperaturen bis zum Siedepunkt des Reaktionsgemisches, mit einem Amin der allgemeinen Formel III umgesetzt wird.

**Claims for the Contracting states: BE, CH, LI, DE, FR, IT, LU, NL, SE**

1. 5-Substituted 5,10-dihydro-11H-dibenzo[b,e] [1,4]diazepin-11-ones of general formula I,

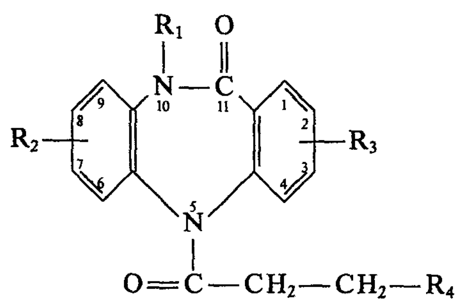

(I)

wherein

$R_1$ represents a hydrogen atom or a methyl or ethyl group;
$R_2$ represents a hydrogen or chlorine atom;
$R_3$ represents a hydrogen or chlorine atom; and
$R_4$ represents a pyrrolidino, piperidino, 2-methyl-piperidino, 2-ethyl-piperidino, 2,6-dimethyl-piperidino or morpholino group;

and the physiologically acceptable salts thereof with inorganic or organic acids.

2. 5-Substituted 5,10-dihydro-11H-dibenzo[b,e] [1,4]diazepin-11-ones of general formula I, as claimed in claim 1, wherein $R_1$ represents a hydrogen atom or a methyl group; $R_2$ and $R_3$ each represents a hydrogen atom; and $R_4$ represents a pyrrolidino or piperidino group; and the physiologically acceptable salts thereof with inorganic or organic acids.

3. 5,10-Dihydro-5-[3-(pyrrolidino)propionyl]-11H-dibenzo[b,e] [1,4]diazepin-11-one and the physiologically acceptable salts thereof with inorganic or organic acids.

4. 5,10-Dihydro-10-methyl-5-[3-(pyrrolidino)propionyl]-11H-dibenzo[b,e] [1,4]diazepin-11-one and the physiologically acceptable salts thereof with inorganic or organic acids.

5. 5,10-Dihydro-5-[3-(piperidino)propionyl]-11H-dibenzo[b,e] [1,4]diazepin-11-one and the physiologically acceptable salts thereof with inorganic or organic acids.

6. Pharmaceutical compositions containing one or more compounds of general formula I as claimed in claim 1, together with the conventional carriers and/or excipients.

7. Use of one or more compounds of general formula I as claimed in claim 1 for the preparation of pharmaceutical compositions by a non-chemical method.

8. Process for the preparation of 5-substituted 5,10-dihydro-11H-dibenzo[b,e] [1,4]diazepin-11-ones of general formula I,

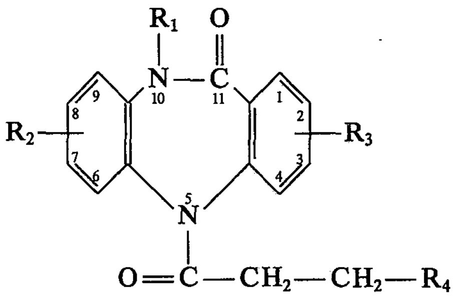

(I)

wherein

$R_1$ represents a hydrogen atom or a methyl or ethyl group;
$R_2$ represents a hydrogen or chlorine atom;
$R_3$ represents a hydrogen or chlorine atom; and
$R_4$ represents a pyrrolidino, piperidino, 2-methyl-piperidino, 2-ethyl-piperidino, 2,6-dimethyl-piperidino or morpholino group;

and the salts thereof with inorganic or organic acids, characterised in that a 5,10-dihydro-11H-dibenzo[b,e] [1,4]diazepin-11-one of general formula II,

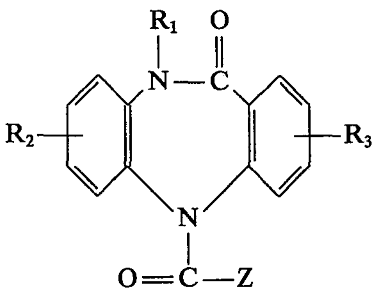

(II)

[wherein $R_1$, $R_2$ and $R_3$ are as hereinbefore defined and Z represents the group $-CH=CH_2$ or $-CH_2-CH_2-Hal$ (in which Hal represents a halogen atom)] is reacted with an amine of general formula III,

$$H-R_4 \qquad (III)$$

(wherein $R_4$ is as hereinbefore defined) and, if desired, a compound of general formula I thus obtained is converted into the salts thereof with inorganic or organic acids.

9. Process as claimed in claim 8, characterised in that a compound of general formula II wherein Z represents the group $-CH_2-CH_2-Hal$ (wherein Hal is a halogen atom, preferably a chlorine or bromine atom) is reacted, in an inert organic solvent at temperatures up to the boiling point of the reaction mixture, with either an at least 2-molar excess of an amine of general formula III or with an amine of general formula III in the presence of a hydrogen halide-binding agent.

10. Process as claimed in claim 8, characterised in that a compound of general formula II wherein Z represents the group $-CH=CH_2$ is reacted with an amine of general formula III in an organic solvent at temperatures up to the boiling point of the reaction mixture.

**Claims for the Contracting state: AT**

1. Process for the preparation of 5-substituted 5,10-dihydro-11H-dibenzo[b,e] [1,4]diazepin-11-ones of general formula I,

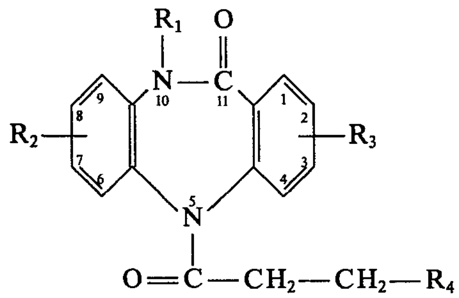

(I)

wherein

$R_1$ represents a hydrogen atom or a methyl or ethyl group;
$R_2$ represents a hydrogen or chlorine atom;
$R_3$ represents a hydrogen or chlorine atom; and
$R_4$ represents a pyrrolidino, piperidino, 2-methyl-piperidino, 2-ethyl-piperidino, 2,6-dimethyl-piperidino or morpholino group;

and the salts thereof with inorganic or organic acids, characterised in that a 5,10-dihydro-11H-dibenzo[b,e] [1,4]diazepin-11-one of general formula II,

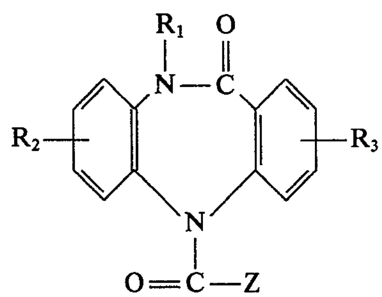

(II)

[wherein $R_1$, $R_2$ and $R_3$ are as hereinbefore defined and Z represents the group $-CH{=}CH_2$ or $-CH_2-CH_2-Hal$ (in which Hal represents a halogen atom)] is reacted with an amine of general formula III,

$$H-R_4 \qquad (III)$$

(wherein $R_4$ is as hereinbefore defined) and, if desired, a compound of general formula I thus obtained is converted into the salts thereof with inorganic or organic acids.

2. Process as claimed in claim 1, characterised in that a compound of general formula II wherein Z represents the group $-CH_2-CH_2-Hal$ (wherein Hal is a halogen atom, preferably a chlorine or bromine atom) is reacted, in an inert organic solvent at temperatures up to the boiling point of the reaction mixture, with either an at least 2-molar excess of an amine of general formula III or with an amine of general formula III in the presence of a hydrogen halide-binding agent.

3. Process as claimed in claim 1, characterised in that a compound of general formula II wherein Z represents the group $-CH{=}CH_2$ is reacted with an amine of general formula III in an organic solvent at temperatures up to the boiling point of the reaction mixture.

**Revendications pour les Etats contractants: BE, CH, LI, DE, FR, IT, LU, NL, SE**

1. 5,10-dihydro-11H-dibenzo[b,e] [1,4]diazépine-11-ones substituées en position 5, de formule générale I:

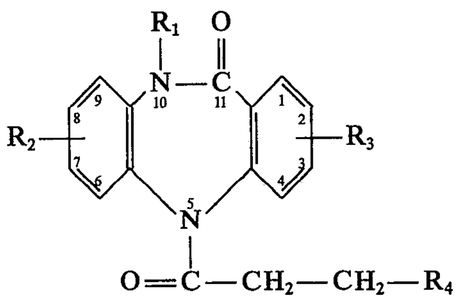

(I)

dans laquelle:

$R_1$ représente un atome d'hydrogène, le groupe méthyle ou éthyle,
$R_2$ représente un atome d'hydrogène ou de chlore,
$R_3$ représente un atome d'hydrogène ou de chlore et
$R_4$ représente le groupe pyrrolidino, pipéridino, 2-méthylpipéridino, 2-éthylpipéridino, 2,6-diméthylpipéridino ou morpholino

et leurs sels physiologiquement supportables avec des acides minéraux ou organiques.

2. 5,10-dihydro-11H-dibenzo[b,e] [1,4]diazépine-11-ones substituées en position 5, de formule générale I selon la revendication 1, caractérisées en ce que:

$R_1$ représente un atome d'hydrogène ou le groupe méthyle,
$R_2$ et $R_3$ représentent des atomes d'hydrogène, et
$R_4$ représente le groupe pyrrolidino ou pipéridino

et leurs sels physiologiquement supportables avec des acides minéraux ou organiques.

3. La 5,10-dihydro-5-[3-(pyrrolidino)propionyl]-11H-dibenzo[b,e] [1,4]diazépine-11-one et ses sels physiologiquement supportables avec des acides minéraux ou organiques.

4. La 5,10-dihydro-10-méthyl-5-[3-(pyrrolidino)propionyl]-11H-dibenzo[b,e] [1,4]diazépine-11-one et ses sels physiologiquement supportables avec des acides minéraux ou organiques.

5. La 5,10-dihydro-5-[3-(pipéridino)propionyl]-11H-dibenzo[b,e] [1,4]diazépine-11-one et ses sels physiologiquement supportables avec des acides minéraux ou organiques.

6. Médicament contenant un ou plusieurs composés de formule générale I selon la revendication 1, conjointement aux excipients et/ou adjuvants habituels.

7. Utilisation d'un ou plusieurs composés de formule générale I selon la revendication 1, pour la préparation de médicaments par voie non chimique.

8. Procédé de préparation de 5,10-dihydro-11H-dibenzo[b,e] [1,4]diazépine-11-ones substituées en position 5, de formule générale I:

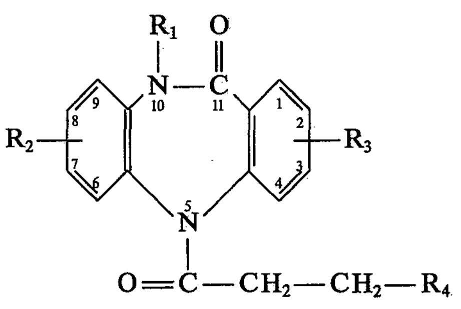

(I)

dans laquelle:

$R_1$ représente un atome d'hydrogène, le groupe méthyle ou éthyle,
$R_2$ représente un atome d'hydrogène ou de chlore,
$R_3$ représente un atome d'hydrogène ou de chlore, et
$R_4$ représente le groupe pyrrolidino, pipéridino, 2-méthyl-pipéridino, 2-éthyl-pipéridino, 2,6-diméthylpipéridino ou morpholino

et de leurs sels avec des acides minéraux ou organiques, caractérisé en ce que: on fait réagir une 5,10-dihydro-11H-dibenzo[b,e] [1,4]diazépine-11-one de formule générale II:

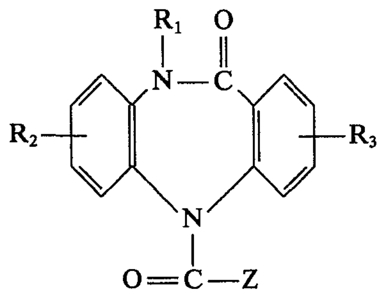

(II)

dans laquelle $R_1$, $R_2$ et $R_3$ sont définis comme plus haut et Z représente le groupe $-CH_2-CH_2-$hal ou $-CH=CH_2$, hal représentant un atome d'halogène, avec une amine de formule générale III:

$$H-R_4 \qquad (III)$$

dans laquelle $R_4$ possède les significations mentionnées plus haut, et éventuellement, on transforme ensuite un composé ainsi obtenu de formule générale I en ses sels au moyen d'acides minéraux ou organiques.

9. Procédé selon la revendication 8, caractérisé en ce que l'on fait réagir un composé de formule générale II dans laquelle Z représente le groupe $-CH_2-CH_2-$hal, hal représentant un atome d'halogène de préférence un atome de chlore ou de brome, dans un solvant organique indifférent, à des températures allant jusqu'à la température d'ébullition du mélange réactionnel, soit avec un excès au moins 2-molaire d'une amine de formule générale III, soit avec une amine de formule générale III en présence d'un agent fixant les halogénures d'acide.

10. Procédé selon la revendication 8, caractérisé en ce que l'on fait réagir un composé de formule générale II dans laquelle Z représente le groupe $-CH=CH_2$, dans un solvant organique, à des températures allant jusqu'à la température d'ébullition du mélange réactionnel, avec une amine de formule générale III.

**Revendications pour l'Etat contractant: AT**

1. Procédé de préparation de 5,10-dihydro-11H-dibenzo[b,e] [1,4]diazépine-11-ones substituées en position 5, de formule générale I:

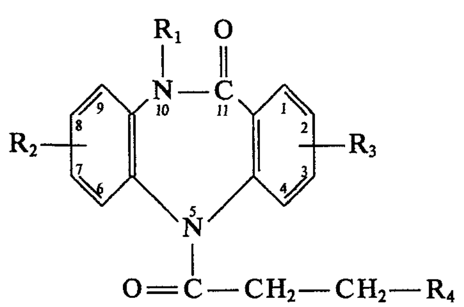

(I)

dans laquelle:

- $R_1$ représente un atome d'hydrogène, le groupe méthyle ou éthyle,
- $R_2$ représente un atome d'hydrogène ou de chlore,
- $R_3$ représente un atome d'hydrogène ou de chlore, et
- $R_4$ représente le groupe pyrrolidino, pipéridino, 2-méthyl-pipéridino, 2-éthyl-pipéridino, 2,6-diméthylpipéridino ou morpholino,

et de leurs sels avec des acides minéraux ou organiques, caractérisé en ce que: on fait réagir une 5,10-dihydro-11H-dibenzo[b,e] [1,4]diazépine-11-one de formule générale II:

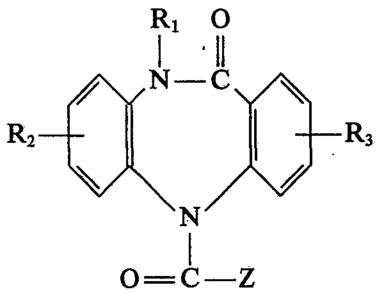

(II)

dans laquelle $R_1$, $R_2$ et $R_3$ sont définis comme plus haut et Z représente le groupe $-CH_2-CH_2-$hal ou $-CH=CH_2$, hal représentant un atome d'halogène, avec une amine de formule générale III:

$$H-R_4 \qquad \text{(III)}$$

dans laquelle $R_4$ possède les significations mentionnées plus haut, et, éventuellement, on transforme ensuite un composé de formule générale I ainsi obtenu en ses sels au moyen d'acides minéraux ou organiques.

2. Procédé selon la revendication 1, caractérisé en ce que l'on fait réagir un composé de formule générale II dans laquelle Z représente le groupe $-CH_2-CH_2-$hal, hal représentant un atome d'halogène, de préférence un atome de chlore ou de brome, dans un solvant organique indifférent à des températures allant jusqu'à la température d'ébullition du mélange réactionnel, avec soit un excès au moins 2 fois molaire d'une amine de formule générale III, soit avec une amine de formule générale III en présence d'un agent fixant les halogénures d'hydrogène.

3. Procédé selon la revendication 1, caractérisé en ce que l'on fait réagir un composé de formule générale II dans laquelle Z représente le groupe $-CH=CH_2$, dans un solvant organique, à des températures allant jusqu'à la température d'ébullition du mélange réactionnel, avec une amine de formule générale III.